(19) Europäisches Patentamt
European Patent Office
Office européen des brevets

(11) EP 3 174 076 A1

(12) **EUROPEAN PATENT APPLICATION**
published in accordance with Art. 153(4) EPC

(43) Date of publication:
**31.05.2017 Bulletin 2017/22**

(21) Application number: **15836717.7**

(22) Date of filing: **27.08.2015**

(51) Int Cl.:
***H01G 9/20*** *(2006.01)*

(86) International application number:
**PCT/JP2015/074258**

(87) International publication number:
**WO 2016/031924 (03.03.2016 Gazette 2016/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **28.08.2014 JP 2014173536**
**28.08.2014 JP 2014173537**

(71) Applicant: **Fujikura Ltd.**
**Tokyo 135-8512 (JP)**

(72) Inventors:
- **MATSUMOTO Daisuke**
**Sakura-shi**
**Chiba 285-8550 (JP)**
- **MATSUI Hiroshi**
**Sakura-shi**
**Chiba 285-8550 (JP)**

(74) Representative: **Hoffmann Eitle**
**Patent- und Rechtsanwälte PartmbB**
**Arabellastraße 30**
**81925 München (DE)**

(54) **ELECTROLYTE FOR DYE-SENSITIZED SOLAR CELL ELEMENTS, AND DYE-SENSITIZED SOLAR CELL ELEMENT USING SAME**

(57) Disclosed is an electrolyte for dye-sensitized solar cell element, which contains a halogen, a halide salt, and a basic substance and in which a redox pair is formed by the halogen and the halide salt, the halogen and the halide salt have the same halogen atom, and the basic substance contains a first imidazole compound constituted by a benzimidazole compound and a second imidazole compound represented by the following formula (1), in which a volume molar concentration $C_2$ of the second imidazole compound is lower than a volume molar concentration $C_1$ of the first imidazole compound:

[Chem. 1]

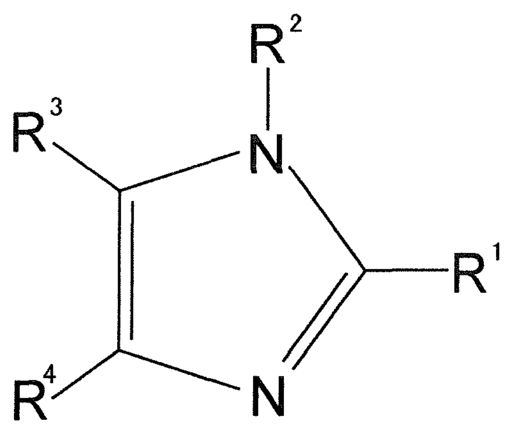

(1)

(in the formula (1), $R^1$ to $R^4$ each independently represent a hydrogen atom, a hydrocarbon group having from 1 to 6 carbon atoms or the like. $R^5$ and $R^6$ each independently represent an aliphatic hydrocarbon group or the like).

# Fig.1

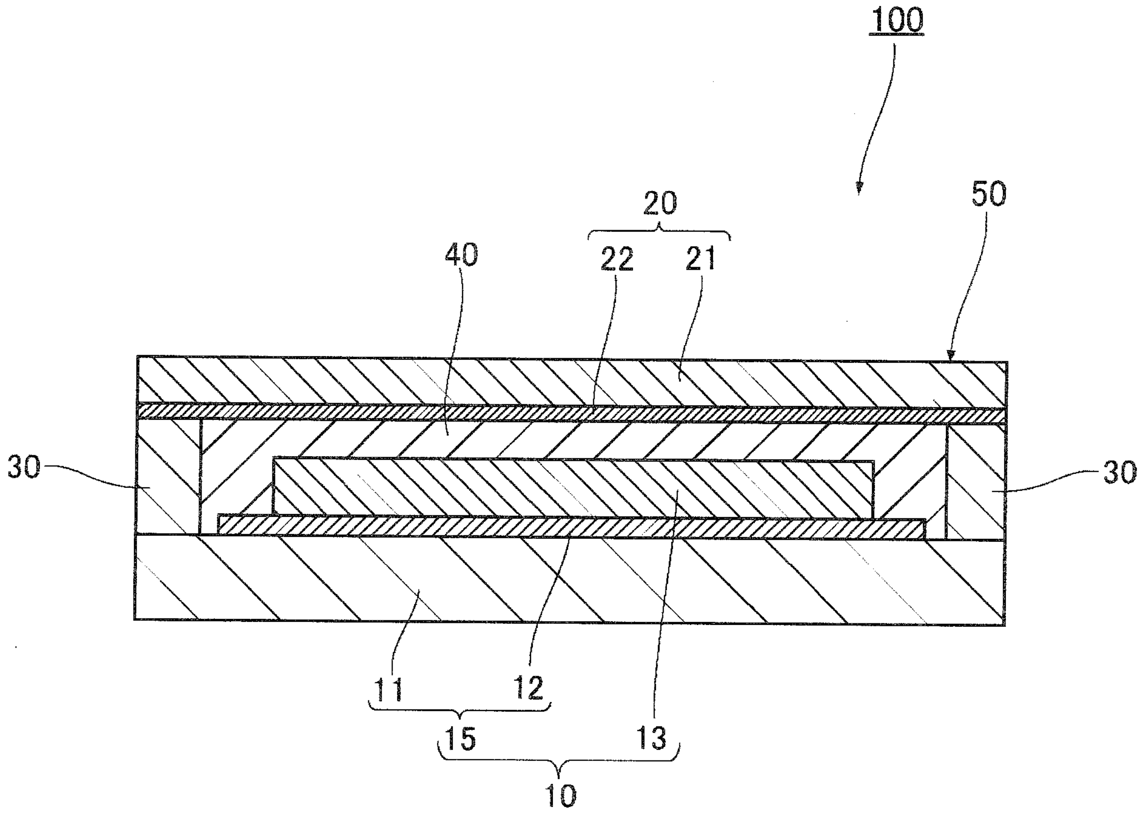

**Description**

TECHNICAL FIELD

The present invention relates to an electrolyte for dye-sensitized solar cell element and a dye-sensitized solar cell element using the same.

BACKGROUND ART

A dye-sensitized solar cell element was developed by Gratzel et al, Switzerland, and it is a next-generation solar cell attracting attention since it has advantages such as a high photoelectric conversion efficiency and low manufacturing cost.

A dye-sensitized solar cell element is generally equipped with a working electrode, a counter electrode, a photosensitizing dye supported on the oxide semiconductor layer of the working electrode, and an electrolyte disposed between the working electrode and the counter electrode. Moreover, the electrolyte contains a redox pair formed, for example, by a halogen and a halide salt.

In a dye-sensitized solar cell element, it is important to improve the photoelectric conversion characteristics, and for this, for example, various proposals which focus on the electrolyte have been made.

For example, in the following Patent Document 1, an electrolyte containing iodine, an iodine compound, and a benzimidazole derivative having a saturated hydrocarbon group that is directly bound to the benzimidazole ring and has from 3 to 11 carbon atoms is disclosed, and it is disclosed that the photoelectric conversion characteristics of a dye-sensitized solar cell element are high and the durability thereof is greatly improved according to this electrolyte.

CITATION LIST

PATENT DOCUMENT

Patent Document 1: JP 2009-2231005 A

SUMMARY OF THE INVENTION

PROBLEM TO BE SOLVED BY THE INVENTION

However, in the dye-sensitized solar cell element that is equipped with an electrolyte described in Patent Document 1, there is still room for improvement in the photoelectric conversion characteristics. Hence, an electrolyte for dye-sensitized solar cell element which can further improve the photoelectric conversion characteristics of the dye-sensitized solar cell element has been desired in the case of being used as an electrolyte of a dye-sensitized solar cell included in a dye-sensitized solar cell element.

The invention has been made in view of the above circumstances, and an object thereof is to provide an electrolyte for dye-sensitized solar cell element which can sufficiently improve the photoelectric conversion characteristics of the dye-sensitized solar cell element and a dye-sensitized solar cell element using the same.

MEANS FOR SOLVING PROBLEM

The present inventors have carried out intensive studies focusing on the composition of the electrolyte in order to solve the above problems. As a result, inventors have noticed realized that a benzimidazole derivative is introduced in order to suppress the leakage current from the oxide semiconductor layer, but it is still insufficient in terms of the ability to suppress the leakage current. Furthermore, the present inventors have found out that it is possible to effectively suppress the leakage current from the oxide semiconductor layer by further blending an imidazole compound that is different from a benzimidazole derivative. Moreover, the present inventors have found out that it is possible to solve the above problems by the following invention as a result of further intensive studies, thereby completing the invention.

In other words, the invention is an electrolyte for dye-sensitized solar cell element, which contains a halogen, a halide salt, and a basic substance and in which a redox pair is formed by the halogen and the halide salt, the halogen and the halide salt have the same halogen atom, and the basic substance contains a first imidazole compound constituted by a benzimidazole compound and a second imidazole compound represented by the following formula (1), in which a volume molar concentration $C_2$ of the second imidazole compound is lower than a volume molar concentration $C_1$ of the first imidazole compound:

[Chem. 1]

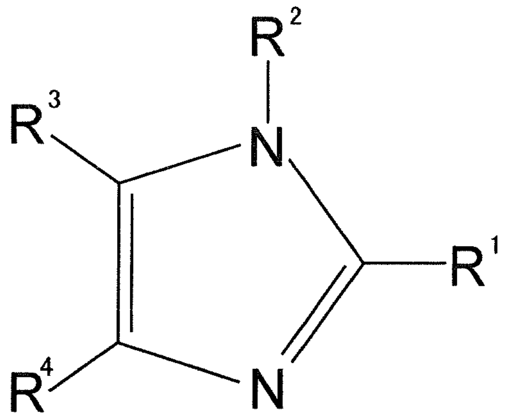

(1)

(in the formula (1), $R^1$ to $R^9$ each independently represent a hydrogen atom, a hydrocarbon group having from 1 to 6 carbon atoms, $-SR^5$, or $-OR^6$. $R^5$ and $R^6$ each independently represent a hydrogen atom or an aliphatic hydrocarbon group).

According to the electrolyte for dye-sensitized solar cell element of the invention, it is possible to sufficiently improve the photoelectric conversion characteristics of a dye-sensitized solar cell element.

The present inventors have presumed the reason for obtaining such an effect as follows. In other words, in a case in which the electrolyte of the invention is used as an electrolyte of a dye-sensitized solar cell included in a dye-sensitized solar cell element having an oxide semiconductor layer composed of an oxide semiconductor, the second imidazole compound in the electrolyte alone significantly improves the open-circuit voltage (Voc) but it correspondingly decreases the short-circuit current (Isc). Accordingly, it is considered that the photoelectric conversion characteristics of a dye-sensitized solar cell element are not improved when the second imidazole compound is blended but the first imidazole compound constituted by a benzimidazole compound is not blended. In addition, the photoelectric conversion characteristics are not sufficiently improved as Isc still decreases when the volume molar concentration of the second imidazole compound is equal to or higher than the volume molar concentration of the first imidazole compound even if the first imidazole compound is blended. However, the photoelectric conversion characteristics are improved when the volume molar concentration of the second imidazole compound is lower than the volume molar concentration of the first imidazole compound. From this fact, the present inventors presumes that the first imidazole compound inhibits the Isc decreasing effect by the second imidazole compound and the photoelectric conversion characteristics of a dye-sensitized solar cell element are improved as a result.

In the electrolyte for dye-sensitized solar cell element, it is preferable that the volume molar concentration $C_2$ of the second imidazole compound be from 0.04 to 0.6 time the volume molar concentration $C_1$ of the first imidazole compound.

In this case, it is possible to more sufficiently improve the photoelectric conversion characteristics of a dye-sensitized solar cell element.

In the electrolyte for dye-sensitized solar cell element, it is preferable that the volume molar concentration $C_1$ of the first imidazole compound be from 30 to 100 mM.

The leakage current can easily be suppressed when $C_1$ is within the above range as compared to a case in which $C_1$ is out of the above range.

In addition, the invention is an electrolyte for dye-sensitized solar cell element, which contains a halogen, a halide salt, and a basic substance and in which a redox pair is formed by the halogen and the halide salt, the halogen and the halide salt have the same halogen atom, and the basic substance contains a first imidazole compound constituted by a benzimidazole compound and a second imidazole compound represented by the following formula (1), in which a molecular weight $M_2$ of the second imidazole compound is smaller than a molecular weight $M_1$ of the first imidazole compound:

[Chem. 2]

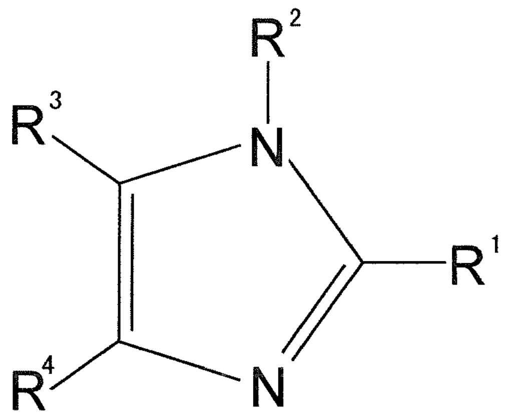

(1)

(in the formula (1), $R^1$ to $R^4$ each independently represent a hydrogen atom, a hydrocarbon group having from 1 to 6 carbon atoms, $-SR^5$, or $-OR^6$. $R^5$ and $R^6$ each independently represent a hydrogen atom or an aliphatic hydrocarbon group).

According to the electrolyte for dye-sensitized solar cell element of the invention, it is possible to sufficiently improve the photoelectric conversion characteristics of a dye-sensitized solar cell element.

The present inventors have presumed the reason for obtaining such an effect as follows. In other words, in a case in which the electrolyte of the invention is used as an electrolyte of a dye-sensitized solar cell included in a dye-sensitized solar cell element having an oxide semiconductor layer composed of an oxide semiconductor, the second imidazole compound in the electrolyte alone significantly improves the open-circuit voltage (Voc) but it correspondingly decreases the short-circuit current (Isc). Accordingly, it is considered that the photoelectric conversion characteristics of a dye-sensitized solar cell element are not improved when the second imidazole compound is blended but the first imidazole compound constituted by a benzimidazole compound is not blended. In addition, the photoelectric conversion characteristics are not sufficiently improved as Isc still decreases when the molecular weight of the second imidazole compound is equal to or more than the molecular weight of the first imidazole compound even if the first imidazole compound is blended. However, the photoelectric conversion characteristics are improved when the molecular weight of the second imidazole compound is smaller than the molecular weight of the first imidazole compound. From this fact, the present inventors presumes that the first imidazole compound inhibits the Isc decreasing effect by the second imidazole compound and thus improves the photoelectric conversion characteristics of a dye-sensitized solar cell element.

In the electrolyte for dye-sensitized solar cell element, it is preferable that the molecular weight $M_2$ of the second imidazole compound is greater than 0 time and 0.67 time or less the molecular weight $M_1$ of the first imidazole compound.

In this case, it is possible to more sufficiently improve the photoelectric conversion characteristics of a dye-sensitized solar cell element.

In the electrolyte for dye-sensitized solar cell element, it is preferable that the molecular weight $M_1$ of the first imidazole compound be from 118 to 224.

The leakage current site can easily be suppressed when $M_1$ is within the above range as compared to a case in which $M_1$ is out of the above range.

In the electrolyte for dye-sensitized solar cell element, it is preferable that the hydrocarbon group is an aliphatic hydrocarbon group in the formula (1).

In this case, it is possible to particularly effectively improve the photoelectric conversion characteristics of the dye-sensitized solar cell element in a case in which the electrolyte is used as an electrolyte of a dye-sensitized solar cell included in a dye-sensitized solar cell element.

In the electrolyte for dye-sensitized solar cell element, it is preferable that the benzimidazole compound constituting the first imidazole compound have a substituent and the substituent be an alkyl group having 2 or more carbon atoms.

In this case, the first imidazole compound can easily cover the leakage current site on the oxide semiconductor layer as compared to a case in which the substituent is an alkyl group having less than the number of carbon atoms or a case in which the benzimidazole compound does not have the substituent.

In addition, the invention is a dye-sensitized solar cell element which includes at least one dye-sensitized solar cell and in which the dye-sensitized solar cell includes a first electrode having a transparent substrate and a transparent conductive film provided on the transparent substrate, a second electrode facing the first electrode, an oxide semiconductor layer provided on the first electrode or the second electrode, an electrolyte provided between the first electrode

and the second electrode, and a photosensitizing dye adsorbed on the oxide semiconductor layer, in which the electrolyte is the electrolyte for dye-sensitized solar cell element described above.

According to the dye-sensitized solar cell element of the invention, it is possible to sufficiently improve the photoelectric conversion characteristics as the electrolyte is composed of the electrolyte for dye-sensitized solar cell element described above.

Incidentally, in the invention, the volume molar concentration of the first imidazole compound refers to the total molar concentration of the volume molar concentrations of the plural kinds of first imidazole compounds in a case in which plural kinds of first imidazole compounds having different volume molar concentrations are contained in the electrolyte.

In addition, the volume molar concentration of the second imidazole compound refers to the total molar concentration of the volume molar concentrations of the plural kinds of second imidazole compounds in a case in which plural kinds of second imidazole compounds having different volume molar concentrations are contained in the electrolyte.

In addition, in the invention, the molecular weight of the first imidazole compound refers to the molecular weight of the first imidazole compound having the smallest molecular weight among the molecular weights of the plural kinds of first imidazole compounds in a case in which plural kinds of first imidazole compounds having different molecular weights are contained in the electrolyte.

In addition, the molecular weight of the second imidazole compound refers to the molecular weight of the second imidazole compound having the largest molecular weight among the molecular weights of the plural kinds of second imidazole compounds in a case in which plural kinds of second imidazole compounds having different molecular weights are contained in the electrolyte.

EFFECT OF THE INVENTION

According to the invention, an electrolyte for dye-sensitized solar cell element which can sufficiently improve the photoelectric conversion characteristics of a dye-sensitized solar cell element and a dye-sensitized solar cell element using the same are provided.

BRIEF DESCRIPTION OF DRAWINGS

Fig. 1 is a cross-sectional diagram illustrating an embodiment of a dye-sensitized solar cell element of the invention.

MODE(S) FOR CARRYING OUT THE INVENTION

Hereinafter, the first embodiment of the dye-sensitized solar cell element of the invention will be described in detail with reference to Fig. 1.

Fig. 1 is a cross-sectional diagram illustrating the first embodiment of a dye-sensitized solar cell element of the invention.

<<First embodiment>>

As illustrated in Fig. 1, a dye-sensitized solar cell element 100 of the first embodiment is constituted by one dye-sensitized solar cell 50, and the dye-sensitized solar cell 50 is equipped with a working electrode 10 which includes a transparent conductive substrate 15, a counter electrode 20 which faces the transparent conductive substrate 15, an annular sealing portion 30 which connects the transparent conductive substrate 15 and the counter electrode 20. An electrolyte 40 is filled in the cell space formed by the transparent conductive substrate 15, the counter electrode 20, and the sealing portion 30.

The counter electrode 20 is equipped with a conductive substrate 21 and a catalyst layer 22 which is provided on the working electrode 10 side of the conductive substrate 21 and contributes to the reduction of the electrolyte 40. In the present embodiment, the second electrode is constituted by the conductive substrate 21.

Meanwhile, the working electrode 10 includes a transparent conductive substrate 15 and at least one oxide semiconductor layer 13 provided on the transparent conductive substrate 15. The transparent conductive substrate 15 is constituted by a transparent substrate 11 and a transparent conductive film 12 provided on the transparent substrate 11. The oxide semiconductor layer 13 is disposed on the inner side of the sealing portion 30. In addition, a photosensitizing dye is adsorbed on the oxide semiconductor layer 13. In the present embodiment, the first electrode is constituted by the transparent conductive substrate 15.

The electrolyte 40 contains a halogen, a halide salt, and a basic substance. In the electrolyte 40, a redox pair is formed by the halogen and the halide salt. Here, the halogen and the halide salt have the same halogen atom.

Moreover, the basic substance contains a first imidazole compound constituted by a benzimidazole compound and a second imidazole compound represented by the following formula (1), and the volume molar concentration $C_2$ of the second imidazole compound is lower than the volume molar concentration $C_1$ of the first imidazole compound.

[Chem. 3]

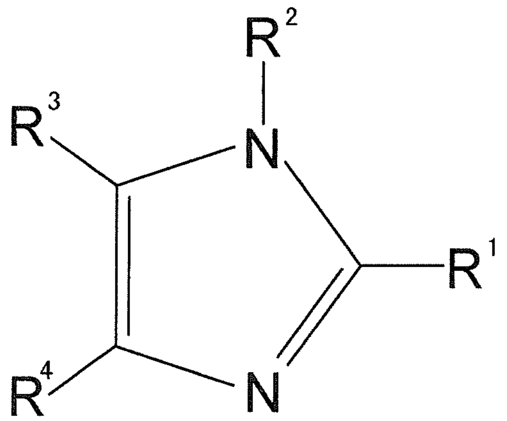

(1)

(in the formula (1), $R^1$ to $R^4$ each independently represent a hydrogen atom, a hydrocarbon group having from 1 to 6 carbon atoms, $-SR^5$, or $-OR^6$. $R^5$ and $R^6$ each independently represent a hydrogen atom or an aliphatic hydrocarbon group).

According to the dye-sensitized solar cell element 100, it is possible to sufficiently improve the photoelectric conversion characteristics as the electrolyte 40 has the configuration described above.

Next, the working electrode 10, the counter electrode 20, the sealing portion 30, the electrolyte 40, and the photosensitizing dye will be described in detail.

<Working electrode>

The working electrode 10 includes the transparent conductive substrate 15 and at least one oxide semiconductor layer 13 provided on the transparent conductive substrate 15 as described above. The transparent conductive substrate 15 is constituted by the transparent substrate 11 and the transparent conductive film 12 provided on the transparent substrate 11.

The material constituting the transparent substrate 11 may be any transparent material, for example, and examples of such a transparent material may include glass such as borosilicate glass, soda lime glass, glass which is made of soda lime and whose iron component is less than that of ordinary soda lime glass, and quartz glass, polyethylene terephthalate (PET), polyethylene naphthalate (PEN), polycarbonate (PC), and polyethersulfone (PES). The thickness of the transparent substrate 11 is appropriately determined depending on the size of the Dye-sensitized solar cell element 100 and is not particularly limited, but it may be set into the range of from 50 to 40000 μm, for example.

Examples of the material constituting the transparent conductive film 12 may include a conductive metal oxide such as indium-tin-oxide (ITO), tin oxide ($SnO_2$), and fluorine-doped-tin-oxide (FTO). The transparent conductive film 12 may be constituted by a single layer or a laminate consisting of a plurality of layers containing different conductive metal oxides. It is preferable that the transparent conductive film 12 contain FTO since FTO exhibits high heat resistance and chemical resistance in a case in which the transparent conductive film 12 is constituted by a single layer. The thickness of the transparent conductive film 12 may be set into the range of from 0.01 to 2 μm, for example.

The oxide semiconductor layer 13 is constituted by oxide semiconductor particles. The oxide semiconductor particles are constituted by, for example, titanium oxide ($TiO_2$), zinc oxide (ZnO, tungsten oxide ($WO_3$), niobium oxide ($Nb_2O_5$), strontium titanate ($SrTiO_3$), tin oxide ($SnO_2$), indium oxide ($In_3O_3$), zirconium oxide ($ZrO_2$), thallium oxide ($Ta_2O_5$), lanthanum oxide ($La_2O_3$), yttrium oxide ($Y_2O_3$), holmium oxide ($Ho_2O_3$), bismuth oxide ($Bi_2O_3$), cerium oxide ($CeO_2$), aluminum oxide ($Al_2O_3$), or two or more kinds of these. The thickness of the oxide semiconductor layer 13 may be set to from 0.1 to 100 μm, for example.

(Counter electrode)

As described above, the counter electrode 20 is equipped with the conductive substrate 21 and the conductive catalyst layer 22 which is provided on the side of the conductive substrate 21 facing the working electrode 10 and which contributes to the reduction of the electrolyte 40.
The conductive substrate 21 is constituted by, for example, a corrosion-resistant metallic material such as titanium, nickel, platinum, molybdenum, tungsten, aluminum and stainless steel or a substrate obtained by forming a film composed of a conductive oxide such as ITO or FTO on the transparent substrate 11 described above. The thickness of the conductive substrate 21 is appropriately determined depending on the size of the dye-sensitized solar cell element 100 and is not particularly limited, but the thickness may be set to from 0.005 to 4 mm, for example.
The catalyst layer 22 is constituted by platinum, a carbon-based material, a conductive polymer or the like. Here, as the carbon-based material, carbon nanotubes are particularly suitably used.

<Sealing portion>

Examples of the sealing portion 30 may include a resin composed of a thermoplastic resin such as a modified polyolefin resin or a vinyl alcohol polymer, and an ultraviolet curable resin. Examples of the modified polyolefin resin may include an ionomer, an ethylene-vinylacetatic anhydride copolymer, an ethylene-methacrylic acid copolymer, and an ethylene-vinyl alcohol copolymer. These resins may be used singly or in combination of two or more kinds thereof.

<Electrolyte>

The electrolyte 40 contains a halogen, a halide salt, and a basic substance as described above. Moreover, in the electrolyte 40, a redox pair is formed by the halogen and the halide salt. In addition, the halogen and the halide salt have the same halogen atom. Moreover, the basic substance contains a first imidazole compound constituted by a benzimidazole compound and a second imidazole compound represented by the following formula (1), and the volume molar concentration $C_2$ of the second imidazole compound is lower than the volume molar concentration $C_1$ of the first imidazole compound, that is, the value of $C_2/C_1$ is less than 1.

(Halogen)

In the electrolyte 40, examples of the halogen atom contained in the halogen and the halide salt may include a bromine atom and an iodine atom.
Among them, the halogen atom contained in the halogen and the halide salt is preferably an iodine atom. In other words, it is preferable that the halogen be iodine and the halide salt be an iodide salt.
In this case, the electron injection efficiency is further improved since the HOMO (bonding orbital) level of the photosensitizing dye used in dye-sensitized solar cell element 100 and the redox level in the electrolyte are at a proper position. In addition, the reduction reaction by iodine is superior to that of other halogen species, thus the electrolyte 40 can instantly return the photoexcited photosensitizing dye to the ground state and it is possible to inhibit the reverse reaction or the like by the photosensitizing dye.

(Halide salt)

Examples of the halide salt contained in the electrolyte 40 may include a bromide salt such as lithium bromide, sodium bromide, potassium bromide, tetramethylammonium bromide, tetraethylammonium bromide, tetrabutylammonium bromide, tetrahexylammonium bromide, 1-hexyl-3-methylimidazolium bromide, 1-ethyl-3-propylimidazolium bromide, 1-ethyl-3-methylimidazolium bromide, 1,2-dimethyl-3-propylimidazolium bromide, 1-butyl-3-methylimidazolium bromide, or 1-methyl-3-propylimidazolium bromide and an iodide salt such as lithium iodide, sodium iodide, potassium iodide, tetramethylammonium iodide, tetraethylammonium iodide, tetrabutylammonium iodide, tetrahexylammonium iodide, 1-hexyl-3-methylimidazolium iodide, 1-ethyl-3-propylimidazolium iodide, 1-ethyl-3-methylimidazolium iodide, 1,2-dimethyl-3-propylimidazolium iodide, 1-butyl-3-methylimidazolium iodide, or 1-methyl-3-propylimidazolium iodide.
Examples of the redox pair formed by the halogen and the halide salt may include a combination of a halide ion and a polyhalide ion. Specific examples thereof may include $I^-/I_3^-$ and $Br^-/Br_3^-$.

(Basic substance)

The first imidazole compound contained in the basic substance is constituted by a benzimidazole compound. The benzimidazole compound may be an unsubstituted benzimidazole compound or a substituted benzimidazole compound having a substituent. Examples of the substituent may include a hydrocarbon group, an ether group, a thiol group, and a nitrile group.

Here, examples of the hydrocarbon group may include an aliphatic hydrocarbon group, an alicyclic hydrocarbon group, and an aromatic hydrocarbon group, and the aliphatic hydrocarbon group is preferable for the reason that the influence of moisture in the electrolyte 40 can be more sufficiently suppressed. The number of carbon atoms in the aliphatic hydrocarbon group is not particularly limited, but it is preferably from 1 to 6. The aliphatic hydrocarbon group may be linear or branched, but it is preferably linear for the reason that the influence of moisture in the electrolyte 40 can be more sufficiently suppressed. Furthermore, the aliphatic hydrocarbon group may be a saturated aliphatic hydrocarbon group or an unsaturated hydrocarbon group, but it is preferably a saturated aliphatic hydrocarbon group. The first imidazole compound hardly reacts with other substances when the aliphatic hydrocarbon group is a saturated aliphatic hydrocarbon group.

Specific examples of the first imidazole compound may include 1-butyl-benzimidazole (NBB), 1-methylbenzimidazole (NMB), 1-ethylbenzimidazole, 1-t-butylbenzimidazole, and 1-hexylbenzimidazole. These may be used singly or in combination of two or more kinds thereof.

The second imidazole compound is represented by the formula (1). In the formula (1), $R^1$ to $R^4$ each independently represent a hydrogen atom, a hydrocarbon group having from 1 to 6 carbon atoms, $-SR^5$, or $-OR^6$. Among them, it is preferable that at least one of $R^1$ to $R^4$ represents a hydrocarbon group having from 1 to 6 carbon atoms, $-SR^5$, or $-OR^6$ for the reason that the second imidazole compound hardly reacts with other substances.

In the formula (1), examples of the hydrocarbon group represented by $R^1$ to $R^4$ may include an aliphatic hydrocarbon group and an aromatic hydrocarbon group. Among them, an aliphatic hydrocarbon group is preferable for the reason that the influence of moisture in the electrolyte 40 can be more sufficiently suppressed. In this case, the number of carbons in the aliphatic hydrocarbon group is not particularly limited, but it is preferably from 1 to 6. In this case, the generation of leakage current at the places to which the photosensitizing dye is not adsorbed on the oxide semiconductor layer 13 can easily be prevented as compared to a case in which the number of carbons is out of this range. The aliphatic hydrocarbon group may be linear or branched, but it is preferably branched. Furthermore, the aliphatic hydrocarbon group may be a saturated aliphatic hydrocarbon group or an unsaturated hydrocarbon group, but it is preferably the saturated aliphatic hydrocarbon group. The second imidazole compound hardly reacts with other substances when the aliphatic hydrocarbon group is a saturated aliphatic hydrocarbon group. Here, the aliphatic hydrocarbon group is preferably an alkyl group having 2 or more carbon atoms. In this case, the first imidazole compound can easily cover the leakage current site on the oxide semiconductor layer 13 as compared to a case in which the substituent is a methyl group or a case in which the benzimidazole compound does not have the substituent.

The number of carbons in the aliphatic hydrocarbon group represented by $R^5$ and $R^6$ is not particularly limited, but it is preferably from 1 to 6 for the reason that the influence of moisture in the electrolyte 40 can be more sufficiently suppressed.

Furthermore, in the formula (1), it is preferable that $R^4$ be a hydrogen atom and at least one of $R^1$ to $R^3$ is a hydrocarbon group having from 1 to 6 carbon atoms, $-SR^5$, or $-OR^6$. In this case, the generation of leakage current can easily be prevented at the places to which the photosensitizing dye is not adsorbed on the oxide semiconductor layer 13.

Specific examples of the second imidazole compound may include 1-methylimidazole (MI), 2-isopropylimidazole (IPI), 1,2-dimethylimidazole, 4-tert-butylimidazole, 1-ethylimidazole-2-thiol, and 1-(tert-butoxycarbonyl)imidazole. These may be used singly or in combination of two or more kinds thereof.

In the electrolyte 40, the volume molar concentration $C_2$ of the second imidazole compound is not particularly limited as long as it is lower than the volume molar concentration $C_1$ of the first imidazole compound, that is, the value of $C_2/C_1$ is less than 1, but the value of $C_2/C_1$ is preferably from 0.04 to 0.6.

In this case, it is possible to particularly sufficiently improve the photoelectric conversion characteristics of the dye-sensitized solar cell element 100 as compared to a case in which the value of $C_2/C_1$ is out of the above range.

The value of $C_2/C_1$ is more preferably from 0.067 to 0.5.

In addition, the volume molar concentration $C_1$ of the first imidazole compound is preferably from 30 to 100 mM.

The leakage current can easily be suppressed when $C_1$ is within the above range as compared to a case in which $C_1$ is out of the above range.

The volume molar concentration $C_1$ of the first imidazole compound is more preferably from 50 to 100 mM.

(Organic solvent)

The electrolyte 40 may further contain an organic solvent. It is possible to use acetonitrile, methoxyacetonitrile, methoxypropionitrile, propionitrile, ethylene carbonate, propylene carbonate, diethyl carbonate, γ-butyrolactone, valeronitrile, pivalonitrile, glutaronitrile, methacrylonitrile, isobutyronitrile, phenyl acetonitrile, acrylonitrile, succinonitrile, oxalonitrile, pentanenitrile, and adiponitrile as the organic solvent. These can be used singly or in combination of two or more kinds thereof.

(Others)

Moreover, as the electrolyte 40, a nanocomposite gel electrolyte of a quasi-solid electrolyte which is obtained by kneading nanoparticles such as $SiO_2$, $TiO_2$, and carbon nanotubes with the electrolyte above to form a gel-like form may be used, or an electrolyte gelled using an organic gelling agent such as polyvinylidene fluoride, a polyethylene oxide derivative, and an amino acid derivative may also be used.

(Photosensitizing dye)

Examples of the photosensitizing dye may include a ruthenium complex having a ligand containing a bipyridine structure or a terpyridine structure, or an organic dye such as porphyrin, eosin, rhodamine, and merocyanine.
Incidentally, it is preferable to use a ruthenium complex having a ligand containing a bipyridine structure as the photosensitizing dye in a case in which the dye-sensitized solar cell element 100 is used indoor or in an environment having a low illumination intensity (10 to 10000 lux).
Next, the method of manufacturing the dye-sensitized solar cell element 100 will be described.
First, a transparent conductive substrate 15 obtained by forming a transparent conductive film on one transparent substrate 11 is prepared.
As the method of forming the transparent conductive film 12, a sputtering method, a vapor deposition method, a spray pyrolysis deposition method (SPD), a CVD method or the like is used.
The paste for oxide semiconductor layer formation contains a resin such as polyethylene glycol and a solvent such as terpineol in addition to the oxide semiconductor particles.
It is possible to use, for example, a screen printing method, a doctor blading method, or a bar coating method as the printing method of the paste for oxide semiconductor layer formation.
The firing temperature varies depending on the kind of the oxide semiconductor particles but is usually from 350 to 600°C, and the firing time also varies depending on the kind of the oxide semiconductor particles but is usually from 1 to 5 hours.
In this manner, the working electrode 10 is obtained.
Next, the photosensitizing dye is supported on the surface of the oxide semiconductor layer 13 of the working electrode 10. For this, the photosensitizing dye may be adsorbed on the oxide semiconductor layer 13 by immersing the working electrode 10 in a solution containing the photosensitizing dye, making the photosensitizing dye adsorb on the oxide semiconductor layer 13 and the extra photosensitizing dye is then washed out with the solvent component of the above solution, and drying is performed, thereby the photosensitizing dye may be adsorbed on the oxide semiconductor layer 13. However, the photosensitizing dye may be adsorbed on the oxide semiconductor layer 13 by coating a solution containing the photosensitizing dye on the oxide semiconductor layer 13 and then drying.
Next, the electrolyte 40 is prepared. The electrolyte 40 is produced by a method including an electrolytic solution preparing step of preparing an electrolytic solution which contains a halogen and a halide salt and has a redox pair formed by the halogen and the halide salt and a basic substance mixing step of obtaining the electrolyte 40 by mixing the electrolytic solution and the basic substance. At this time, in the electrolytic solution preparing step, the halogen and the halide salt are prepared so as to have the same halogen atom. In addition, in the basic substance mixing step, the electrolytic solution and the basic substance are mixed such that the volume molar concentration $C_2$ of the second imidazole compound in the electrolyte 40 to be obtained is lower than the volume molar concentration $C_1$ of the first imidazole compound in the electrolyte 40.
Hereinafter, the method for producing the electrolyte 40 will be specifically described.

(Electrolytic solution preparing step)

First, for example, a solution containing a redox pair formed by a halogen and a halide salt is prepared by dissolving the halogen and the halide salt in the organic solvent described above. In this manner, an electrolytic solution containing the redox pair formed by the halogen and the halide salt is obtained.

(Basic substance mixing step)

Next, the electrolytic solution produced in the manner described above and the basic substance described above are mixed. Here, the basic substance is mixed such that the value of $C_2/C_1$ is less than 1.
In this manner, the electrolyte 40 is obtained. According to the method for producing the electrolyte 40 described above, the electrolyte 40 to be obtained can sufficiently improve the photoelectric conversion characteristics of the dye-sensitized solar cell element 100.
In the basic substance mixing step, it is preferable to mix the electrolytic solution and the basic substance such

that the volume molar concentration $C_2$ of the second imidazole compound is from 0.04 to 0.6 time the volume molar concentration $C_1$ of the first imidazole compound.

In this case, it is possible to more sufficiently improve the photoelectric conversion characteristics of the dye-sensitized solar cell element 100 by the electrolyte 40 to be obtained in this manner.

Here, it is more preferable to mix the electrolytic solution and the basic substance such that the value of $C_2/C_1$ is from 0.067 to 0.5.

In addition, it is preferable to mix the electrolytic solution and the basic substance such that the volume molar concentration $C_1$ of the first imidazole compound is from 30 to 100 mM.

The electrolyte 40 can more sufficiently improve the photoelectric conversion characteristics of the dye-sensitized solar cell element 100 when $C_1$ is within the above range as compared to a case in which $C_1$ is out of the above range.

At this time, it is more preferable to mix the electrolytic solution and the basic substance such that $C_1$ is from 50 to 100 mM.

Next, the electrolyte 40 is disposed on the oxide semiconductor layer 13. The electrolyte 40 can be disposed, for example, by a printing method such as screen printing.

Next, an annular sealing portion forming body is prepared. The sealing portion forming body can be obtained, for example, by preparing a resin film for sealing and forming one quadrangular opening in the resin film for sealing.

Thereafter, this sealing portion forming body is bonded onto the working electrode 10. In this time, bonding of the sealing portion forming body to the working electrode 10 can be conducted, for example, by melting and heating the sealing portion forming body.

Next, the counter electrode 20 is prepared, disposed so as to close the opening of the sealing portion forming body, and then bonded to the sealing portion forming body. At this time, the sealing portion forming body may be bonded to the counter electrode 20 in advance and this sealing portion forming body may be bonded to the sealing portion forming body on the working electrode 10 side. Bonding of the sealing portion forming body to the counter electrode 20 may be conducted under the atmospheric pressure or reduced pressure, but it is preferably conducted under reduced pressure.

The dye-sensitized solar cell element 100 is obtained in the manner described above.

<<Second embodiment>>

Next, the second embodiment of the dye-sensitized solar cell element of the invention will be described.

The dye-sensitized solar cell element according to the second embodiment differs from the dye-sensitized solar cell element 100 of the first embodiment in the electrolyte 40. Specifically, the molecular weight $M_2$ of the second imidazole compound is smaller than the molecular weight $M_1$ of the first imidazole compound in the dye-sensitized solar cell element of the second embodiment, while the volume molar concentration $C_2$ of the second imidazole compound is lower than the volume molar concentration $C_1$ of the first imidazole compound in the dye-sensitized solar cell element 100 of the first embodiment. It is the same as the electrolyte 40 in the dye-sensitized solar cell element of the first embodiment that the electrolyte 40 in the dye-sensitized solar cell element of the second embodiment contains a halogen, a halide salt, and a basic substance, a redox pair is formed by the halogen and the halide salt, the halogen and the halide salt have the same halogen atom, and the basic substance contains a first imidazole compound constituted by a benzimidazole compound and a second imidazole compound represented by the following formula (1).

According to the dye-sensitized solar cell element according to the second embodiment, it is possible to sufficiently improve the photoelectric conversion characteristics as the electrolyte 40 has the configuration described above.

In the electrolyte 40, the molecular weight $M_2$ of the second imidazole compound is not particularly limited as long as it is smaller than the molecular weight $M_1$ of the first imidazole compound, that is, the value of $M_2/M_1$ is less than 1, but the value of $M_2/M_1$ is preferably greater than 0 and 0.67 or less.

In this case, it is possible to particularly sufficiently improve the photoelectric conversion characteristics of the dye-sensitized solar cell element 100 as compared to a case in which the value of $M_2/M_1$ is out of the above range.

The value of $M_2/M_1$ is more preferably from 0.3 to 0.63 and particularly preferably from 0.47 to 0.63.

In addition, the molecular weight $M_1$ of the first imidazole compound is preferably from 118 to 224.

The leakage current site can easily be suppressed when $M_1$ is within the above range as compared to a case in which $M_1$ is out of the above range.

$M_1$ is more preferably from 130 to 210.

Next, a method for producing the dye-sensitized solar cell element of the second embodiment will be described. The method for producing the dye-sensitized solar cell element of the second embodiment differs from the method for producing the dye-sensitized solar cell element 100 of the first embodiment only in the method for producing the electrolyte 40. Accordingly, hereinafter, the method for producing the electrolyte 40 in the dye-sensitized solar cell element of the second embodiment will be described.

The electrolyte 40 is produced by a method including an electrolytic solution preparing step of preparing an

electrolytic solution which contains a halogen and a halide salt and has a redox pair formed by the halogen and the halide salt and a basic substance mixing step of obtaining the electrolyte 40 by mixing the electrolytic solution and the basic substance. At this time, in the electrolytic solution preparing step, the halogen and the halide salt are prepared so as to have the same halogen atom. In addition, in the basic substance mixing step, the electrolytic solution and the basic substance are mixed such that the molecular weight $M_2$ of the second imidazole compound in the electrolyte 40 to be obtained is smaller than the molecular weight $M_1$ of the first imidazole compound in the electrolyte 40.

Hereinafter, the method for producing the electrolyte 40 will be specifically described.

(Electrolytic solution preparing step)

First, for example, a solution containing a redox pair formed by a halogen and a halide salt is prepared by dissolving the halogen and the halide salt in an organic solvent. In this manner, an electrolytic solution containing the redox pair formed by the halogen and the halide salt is obtained.

(Basic substance mixing step)

Next, the electrolytic solution produced in the manner described above and the basic substance described above are mixed. Here, the basic substance is mixed such that the value of $M_2/M_1$ is less than 1.

In this manner, the electrolyte 40 is obtained. According to the method for producing the electrolyte 40 described above, the electrolyte 40 to be obtained can sufficiently improve the photoelectric conversion characteristics of the dye-sensitized solar cell element 100.

In the basic substance mixing step, it is preferable to mix the electrolytic solution and the basic substance such that the molecular weight $M_2$ of the second imidazole compound is greater than 0 time and 0.67 time or less the molecular weight $M_1$ of the first imidazole compound.

In this case, it is possible to more sufficiently improve the photoelectric conversion characteristics of dye-sensitized solar cell element 100 by the electrolyte 40 to be obtained in this manner.

Here, it is more preferable to mix the electrolytic solution and the basic substance such that the value of $M_2/M_1$ is preferably from 0.3 to 0.63 and more preferably from 0.47 to 0.6.

In addition, it is preferable to mix the electrolytic solution and the basic substance such that the molecular weight $M_1$ of the first imidazole compound is from 118 to 224.

The electrolyte 40 can more sufficiently improve the photoelectric conversion characteristics of the dye-sensitized solar cell element 100 when $M_1$ is within the above range as compared to a case in which $M_1$ is out of the above range.

At this time, it is more preferable to mix the electrolytic solution and the basic substance such that $M_1$ is from 130 to 210.

The invention is not limited to the embodiments described above. For example, in the embodiments described above, the dye-sensitized solar cell element 100 has a structure in which the porous oxide semiconductor layer 13 is provided on the transparent conductive film 12 of the transparent conductive substrate 15 and light is thus received from the transparent conductive substrate 15 side, but it may have a structure in which an opaque material (for example, metal substrate) is used as the base material on which the porous oxide semiconductor layer 13 is formed, a transparent material is used as a base material for forming the counter electrode 20, and light is thus received from the counter electrode side, and further, it may have a structure in which light is received from both surfaces.

In addition, in the embodiments described above, the dye-sensitized solar cell element is constituted by one dye-sensitized solar cell 50, but the dye-sensitized solar cell element may be equipped with a plurality of dye-sensitized solar cells 50.

## EXAMPLES

Hereinafter, the contents of the invention will be more specifically described with reference to Examples, but the invention is not limited to the following Examples.

(Examples 1A to 12A and Comparative Examples 1A to 4A)

<Preparation of electrolyte for dye-sensitized solar cell element>

An electrolytic solution was prepared by dissolving the first imidazole compound and the second imidazole compound presented in the following Table 1 in a mixture of 0.002 g of iodine, 3.1 g of 1,2-dimethyl-3-propylimidazolium iodide (DMPImI), and 20 mL of 3-methoxypropionitrile. Here, "NBB", "MI", and "IPI" described in Table 1 are as follows.

NBB: 1-butylbenzimidazole
MI: 1-methylimidazole
IPI: 2-isopropylimidazole

At this time, the first imidazole compound and the second imidazole compound were added such that the volume molar concentrations $C_1$ and $C_2$ in the electrolyte and the volume molar concentration ratio ($C_2/C_1$) in the electrolyte were the values presented in Table 1. The electrolyte for dye-sensitized solar cell element was prepared in this manner.

(Examples 1B and 2B and Comparative Examples 1B to 6B)

<Preparation of electrolyte for dye-sensitized solar cell element>

An electrolytic solution was prepared by dissolving the first imidazole compound and the second imidazole compound presented in the following Table 2 in a mixture of 0.002 g of iodine, 3.1 g of 1,2-dimethyl-3-propylimidazolium iodide (DMPImI), and 20 mL of 3-methoxypropionitrile. Here, "NBB", "NMB", "MI", and "IPI" described in Table 2 are as follows.

NBB: 1-butylbenzimidazole
NMB: 1-methylbenzimidazole
MI: 1-methylimidazole
IPI: 2-isopropylimidazole

At this time, the molecular weights $M_1$ and $M_2$ of the first imidazole compound and the second imidazole compound and the molecular weight ratio ($M_2/M_1$) thereof in the electrolyte were as presented in Table 2. In addition, the volume molar concentration of the first imidazole compound in the electrolyte was set to 0.1 M in the case of blending the first imidazole compound in the electrolyte. Furthermore, the volume molar concentration of the second imidazole compound in the electrolyte was set to 0.01 M in the case of blending the second imidazole compound in the electrolyte. The electrolyte for dye-sensitized solar cell element was prepared in this manner.

<Fabrication of dye-sensitized solar cell element>

First, an FTO/glass substrate obtained by forming an FTO film on a glass substrate was prepared. Thereafter, this FTO/glass substrate was washed, subjected to the UV-$O_3$ treatment, coated with a titanium oxide nanoparticle paste containing titanium oxide by screen printing to form a film of 50×50 mm on the substrate, and dried for 10 minutes at 150°C. In this manner, a non-calcined substrate was obtained. Thereafter, this non-calcined substrate was put in an oven, and the titanium oxide nanoparticle paste was calcined for 1 hour at 500°C to form a porous titanium oxide layer having a thickness of 14 μm on the FTO film, thereby obtaining a working electrode.

Next, a dye solution was prepared by dissolving the Z907 photosensitizing dye in a mixed solvent prepared by mixing acetonitrile and t-butyl alcohol at 1 : 1 (volume ratio). Thereafter, the working electrode was immersed in this dye solution for 24 hours to support the photosensitizing dye on the porous titanium oxide layer.

Meanwhile, the FTO/glass substrate used in the fabrication of the working electrode was prepared, and Pt was deposited on this substrate by a sputtering method. The counter electrode was obtained in this manner.

Next, an annular thermoplastic resin sheet composed of the Himilan (trade name, manufactured by DU PONT-MITSUI POLYCHEMICALS CO., LTD.) of an ionomer was disposed on the working electrode. At this time, the porous titanium oxide layer was disposed on the inner side of the annular thermoplastic resin sheet. Thereafter, the thermoplastic resin sheet was melted by being heated for 5 minutes at 180°C and bonded to the working electrode.

Meanwhile, the electrolyte prepared in the manner described above was coated on the working electrode by a screen printing method so as to cover the porous titanium oxide layer.

Thereafter, the counter electrode was superimposed on the working electrode so as to sandwich the electrolyte between the counter electrode and the working electrode, and the sealing portion was melted and heated under reduced pressure (1000 Pa) to bond the counter electrode and the sealing portion.

In this manner, a dye-sensitized solar cell element was obtained.

<Evaluation of characteristics>

(Photoelectric conversion characteristics)

The photoelectric conversion efficiency η (%) of the dye-sensitized solar cell elements of Examples 1A to 12A and Comparative Examples 1A to 4A obtained in the manner described above was measured. Thereafter, the rate of increase in η was calculated based on the following Equation by taking Comparative Example 1A as the criterion comparative example. The results thereof are presented in Table 1.

$$\text{Rate of increase in } \eta\ (\%) = 100 \times (\eta \text{ of Example or Comparative Example} - \eta \text{ of Comparative Example 1A})/\eta \text{ of Comparative Example 1A}$$

At this time, the measurement of η was conducted by using the Xe lamp solar simulator (YSS-150 manufactured by Yamashita Denso Corporation) and the IV tester (MP-160 manufactured by EKO Instruments).

In addition, the photoelectric conversion efficiency η (%) of the dye-sensitized solar cell elements of Examples 1B and 2B and Comparative Examples 1B to 6B obtained in the manner described above was measured. Thereafter, the rate of increase in η was calculated based on the following Equation by taking Comparative Example 1B as the criterion comparative example. The results thereof are presented in Table 2.

$$\text{Rate of increase in } \eta\ (\%) = 100 \times (\eta \text{ of Example or Comparative Example} - \eta \text{ of Comparative Example 1B})/\eta \text{ of Comparative Example 1B}$$

At this time, the measurement of η was conducted by using the Xe lamp solar simulator (YSS-150 manufactured by Yamashita Denso Corporation) and the IV tester (MP-160 manufactured by EKO Instruments) in the same manner as in Example 1A.

[Table 1]

| | First imidazole compound | | Second imidazole compound | | $C_2/C_1$ | Photoelectric conversion characteristics |
|---|---|---|---|---|---|---|
| | Kind | Volume molar concentration $C_1$ (mM) | Kind | Volume molar concentration $C_2$ (mM) | | Rate of increase in η (%) |
| Example 1A | NBB | 30 | MI | 2 | 0.067 | 11 |
| Example 2A | NBB | 100 | MI | 10 | 0.1 | 13 |
| Example 3A | NBB | 100 | MI | 20 | 0.2 | 13 |
| Example 4A | NBB | 30 | MI | 10 | 0.33 | 12 |
| Example 5A | NBB | 80 | MI | 40 | 0.5 | 6 |
| Example 6A | NBB | 30 | IPI | 2 | 0.067 | 13 |
| Example 7A | NBB | 100 | IPI | 10 | 0.1 | 14 |
| Example 8A | NBB | 100 | IPI | 20 | 0.2 | 15 |
| Example 9A | NBB | 30 | MI | 10 | 0.33 | 13 |
| Example 10A | NBB | 80 | MI | 40 | 0.5 | 8 |
| Example 11A | NBB | 30 | IPI | 1 | 0.033 | 4 |

(continued)

| | First imidazole compound | | Second imidazole compound | | $C_2/C_1$ | Photoelectric conversion characteristics |
|---|---|---|---|---|---|---|
| | Kind | Volume molar concentration $C_1$ (mM) | Kind | Volume molar concentration $C_2$ (mM) | | Rate of increase in η (%) |
| Example 12A | NBB | 30 | IPI | 20 | 0.67 | 2 |
| Comparative Example 1A | - | 0 | - | 0 | - | - |
| Comparative Example 2A | NBB | 120 | IPI | 120 | 1 | -4 |
| Comparative Example 3A | NBB | 120 | IPI | 240 | 2 | -8 |
| Comparative Example 4A | NBB | 120 | IPI | 600 | 5 | -17 |

[Table 2]

| | First imidazole compound | | Second imidazole compound | | $M_2/M_1$ | Photoelectric conversion characteristics |
|---|---|---|---|---|---|---|
| | Kind | Molecular weight $M_1$ | Kind | Molecular weight $M_2$ | | Rate of increase in η (%) |
| Example 1B | NBB | 174 | MI | 82 | 0.47 | 11 |
| Example 2B | NBB | 174 | IPI | 110 | 0.63 | 18 |
| Comparative Example 1B | - | - | - | - | - | 0 |
| Comparative Example 2B | NBB | 174 | - | - | 0 | 8 |
| Comparative Example 3B | NMB | 132 | - | - | 0 | 5 |
| Comparative Example 4B | - | - | MI | 82 | - | -10 |
| Comparative Example 5B | - | - | IPI | 110 | - | -9 |
| Comparative Example 6B | - | - | MI+IPI | 110 | - | -15 |

From the results presented in Table 1, it was found that the rate of increase in η of the dye-sensitized solar cell elements of Examples 1A to 12A obtained by taking Comparative Example 1A as the criterion is 2% or more. On the other hand, it was found that the rate of increase in η of the dye-sensitized solar cell elements of Comparative Examples 2A to 4A obtained by taking Comparative Example 1A as the criterion is -4% or less.

From the results presented in Table 2, it was found that the rate of increase in η of the dye-sensitized solar cell elements of Examples 1B and 2B obtained by taking Comparative Example 1B as the criterion is 11% or more. On the other hand, it was found that the rate of increase in η of the dye-sensitized solar cell elements of Comparative Examples 2B to 6B obtained by taking Comparative Example 1B as the criterion is 8% or less.

As described above, it was confirmed that it is possible to sufficiently improve the photoelectric conversion characteristics of a dye-sensitized solar cell element according to the electrolyte for dye-sensitized solar cell element of the invention.

EXPLANATIONS OF REFERRENCE NUMERALS

11 ... Transparent substrate
12 ... Transparent conductive film
13 ... Oxide semiconductor layer
15 ... Transparent conductive substrate (first electrode)
20 ... Counter electrode (second electrode)
40 ... Electrolyte
50 ... Dye-sensitized solar cell
100 ... Dye-sensitized solar cell element

## Claims

**1.** An electrolyte for dye-sensitized solar cell element comprising:

a halogen;
a halide salt; and
a basic substance, wherein
a redox pair is formed by the halogen and the halide salt,
the halogen and the halide salt have the same halogen atom, and
the basic substance contains a first imidazole compound constituted by a benzimidazole compound and a second imidazole compound represented by the following formula (1), wherein
a volume molar concentration $C_2$ of the second imidazole compound is lower than a volume molar concentration $C_1$ of the first imidazole compound:

[Chem. 1]

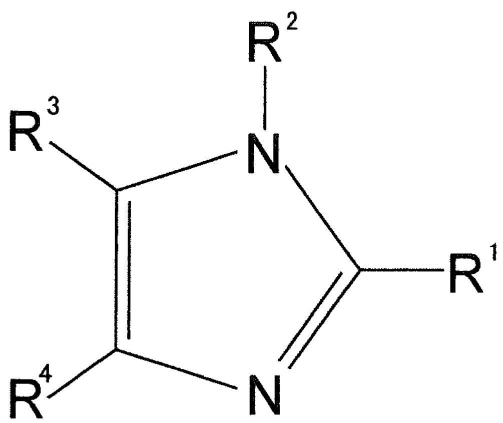

(1)

(in the formula (1), $R^1$ to $R^4$ each independently represent a hydrogen atom, a hydrocarbon group having from 1 to 6 carbon atoms, $-SR^5$, or $-OR^6$. $R^5$ and $R^6$ each independently represent a hydrogen atom or an aliphatic hydrocarbon group).

**2.** The electrolyte for dye-sensitized solar cell element according to claim 1, wherein the volume molar concentration $C_2$ of the second imidazole compound is from 0.04 to 0.6 time the volume molar concentration $C_1$ of the first imidazole compound.

**3.** The electrolyte for dye-sensitized solar cell element according to claim 1 or 2, wherein the volume molar concentration $C_1$ of the first imidazole compound is from 30 to 100 mM.

**4.** An electrolyte for dye-sensitized solar cell element comprising:

a halogen;
a halide salt; and
a basic substance, wherein
a redox pair is formed by the halogen and the halide salt,
the halogen and the halide salt have the same halogen atom, and
the basic substance contains a first imidazole compound constituted by a benzimidazole compound and a second imidazole compound represented by the following formula (1), wherein
a molecular weight $M_2$ of the second imidazole compound is smaller than a molecular weight $M_1$ of the first imidazole compound:

[Chem. 2]

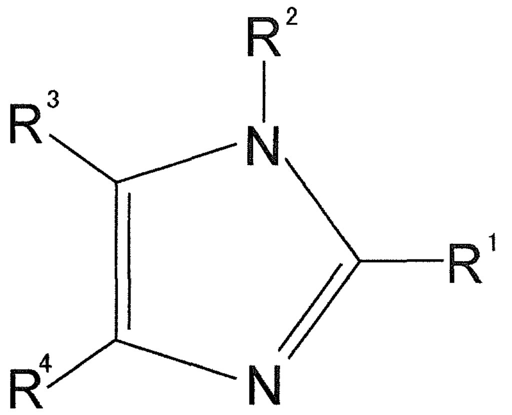

(1)

(in the formula (1), $R^1$ to $R^4$ each independently represent a hydrogen atom, a hydrocarbon group having from 1 to 6 carbon atoms, $-SR^5$, or $-OR^6$. $R^5$ and $R^6$ each independently represent a hydrogen atom or an aliphatic hydrocarbon group).

5. The electrolyte for dye-sensitized solar cell element according to claim 4, wherein the molecular weight $M_2$ of the second imidazole compound is greater than 0 time and 0.67 time or less the molecular weight $M_1$ of the first imidazole compound.

6. The electrolyte for dye-sensitized solar cell element according to claim 4 or 5, wherein the molecular weight $M_1$ of the first imidazole compound is from 118 to 224.

7. The electrolyte for dye-sensitized solar cell element according to any one of claims 1 to 6, wherein the hydrocarbon group is an aliphatic hydrocarbon group in the formula (1).

8. The electrolyte for dye-sensitized solar cell element according to any one of claims 1 to 7, wherein the hydrocarbon group is an aliphatic hydrocarbon group in the formula (1).

9. The electrolyte for dye-sensitized solar cell element according to any one of claims 1 to 8, wherein the halogen atom is iodine.

10. A dye-sensitized solar cell element comprising at least one dye-sensitized solar cell, wherein
the dye-sensitized solar cell includes
a first electrode having a transparent substrate and a transparent conductive film provided on the transparent substrate,
a second electrode facing the first electrode,
an oxide semiconductor layer provided on the first electrode or the second electrode,
an electrolyte provided between the first electrode and the second electrode, and
a photosensitizing dye adsorbed on the oxide semiconductor layer, wherein
the electrolyte includes the electrolyte for dye-sensitized solar cell element according to any one of claims 1 to 9.

Fig.1

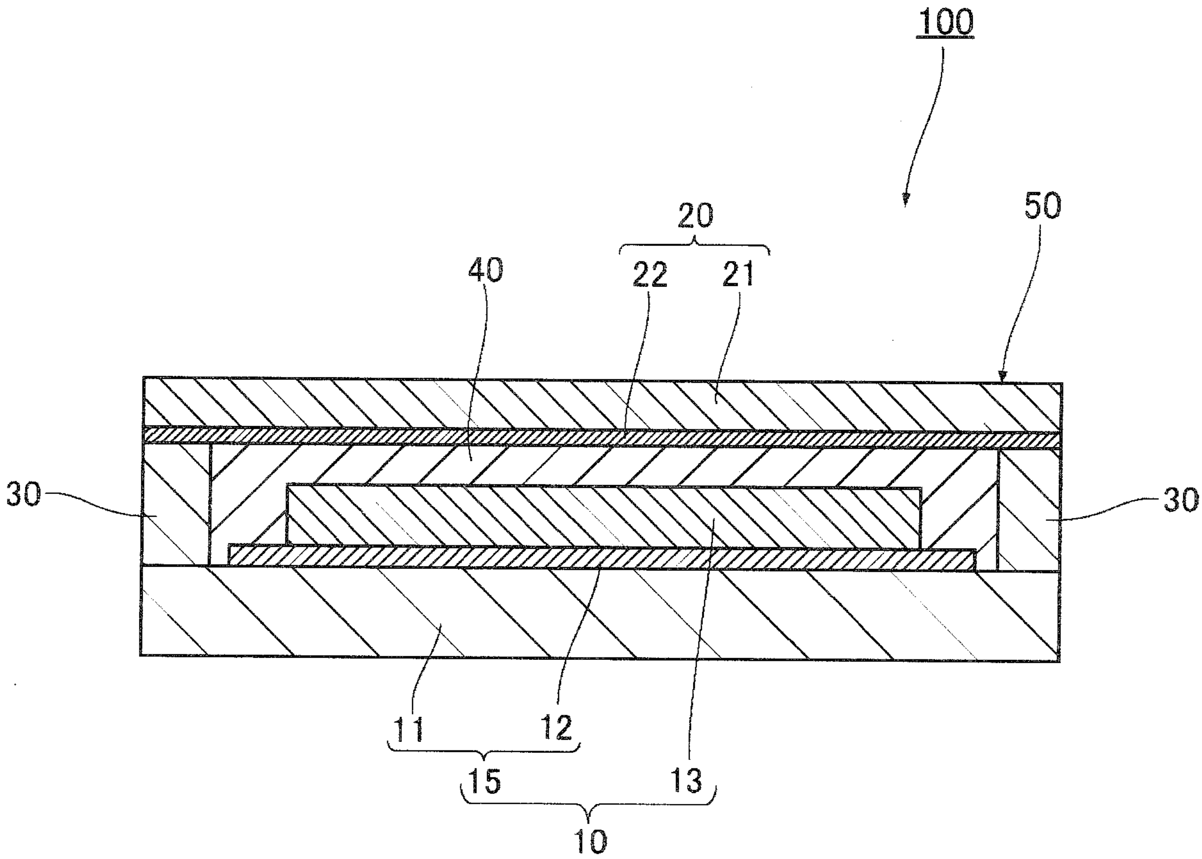

## INTERNATIONAL SEARCH REPORT

International application No. PCT/JP2015/074258

A. CLASSIFICATION OF SUBJECT MATTER
*H01G9/20*(2006.01)i

According to International Patent Classification (IPC) or to both national classification and IPC

B. FIELDS SEARCHED

Minimum documentation searched (classification system followed by classification symbols)
H01G9/20

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 2012-28298 A (Peccell Technologies, Inc.), 09 February 2012 (09.02.2012), claims; examples (Family: none) | 1-4,6-10<br>5 |
| A | JP 2012-144688 A (Fujifilm Corp.), 02 August 2012 (02.08.2012), claims; examples & WO 2011/152284 A1 & TW 201210835 A & CN 102906935 A & KR 10-2013-0086943 A | 1-10 |
| A | JP 2013-69557 A (Osaka Gas Co., Ltd.), 18 April 2013 (18.04.2013), claims; examples (Family: none) | 1-10 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30 September 2015 (30.09.15) | 13 October 2015 (13.10.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

International application No.
PCT/JP2015/074258

C (Continuation). DOCUMENTS CONSIDERED TO BE RELEVANT

| Category* | Citation of document, with indication, where appropriate, of the relevant passages | Relevant to claim No. |
|---|---|---|
| A | JP 2006-331995 A (Sharp Corp.), 07 December 2006 (07.12.2006), claims; examples (Family: none) | 1-10 |
| A | US 2014/0190567 A1 (MERCK PATENT GMBH), 10 July 2014 (10.07.2014), claims; examples & JP 2014-529895 A & WO 2013/026563 A1 | 1-10 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 20092231005 A